# EUROPEAN PATENT APPLICATION

(11) **EP 4 086 340 A1**
(43) Date of publication of application: **09.11.2022**
(21) Application number: 20910719.2
(22) Date of filing: 28.12.2020
(51) Int. Cl.: C12N 5/10, C12N 15/113

(54) **UNIVERSAL CAR-T TARGETING T-CELL LYMPHOMA CELL AND PREPARATION METHOD THEREFOR AND USE THEREOF**

(30) Priority: 30.12.2019 WO PCT/CN2019/129956
(71) Applicant: Edigene Biotechnology Inc., Beijing 102206 (CN)
(72) Inventor: WANG, Fei, Beijing 102206 (CN); YUAN, Pengfei, Beijing 102206 (CN); NIU, Lichao, Beijing 102206 (CN); FU, Jianqiang, Beijing 102206 (CN); WU, Lanlan, Beijing 102206 (CN)
(74) Representative: Lavoix
(86) International application number: PCT/CN2020/140176
(87) International publication number: WO 2021/136176

(57) **Abstract**

A novel preparation method for a universal CAR-T cell targeting a T-cell lymphoma cell, the universal CAR-T cell prepared by means of the method and a biological product comprising the universal CAR-T cell. The preparation method for the universal CAR-T cell targeting a T-cell lymphoma cell comprises: obtaining a T cell from a human donor having a healthy lymphatic system, and then disrupting a TRAC genome region and a B2M genome region in the T cell by means of a gene editing technology, so that CAR molecules targeting the T-cell lymphoma cell TCRα/β are stably expressed in the T cell. The universal CAR-T cell targeting a T-cell lymphoma cell prepared by means of the preparation method eliminates the natural TCR expression of a T cell, greatly reduces the graft-versus-host reaction, and meanwhile, also greatly reduces the immunogenicity thereof, and can continuously and efficiently kill the T-cell lymphoma cell.

## Description

### TECHNICAL FIELD

The present invention relates to the preparation technology of universal CAR-T for treating T-cell lymphoma, the universal CAR-T cell prepared by the preparation technology and use thereof.

### BACKGROUND ART

Malignant tumors have become a serious threat to health and life safety, and the cure of tumors has always been the dream of mankind. In recent years, tumor immunotherapy has gained extensive attention; especially the emergence of CAR-T (chimeric antigen receptor T cells) technology has made a milestone development in tumor control. From the first application of CAR-T technology in 1989, to Emily Whitehead, who was cured by a team led by Professor Carl June of the University of Pennsylvania in 2012, and to the approval of Novartis CAR-T drug CTL019 for treating juvenile advanced B-cell acute lymphoblastic leukemia (r/rAll) unanimously recommended by the FDA Oncology Drug Expert Advisory Committee (ODAC) in 2017 with an overwhelming vote of 10:0.

Generally, T cells in traditional CAR-T technology are mainly derived from a patient per se. In a GMP environment, T cells are isolated in vitro, activated, CAR introduced, cultured and expanded, and finally returned to the patient after quality control. However, since the T cells of a patient with T-lineage lymphocytic leukemia are cancerous, they cannot be used for CAR-T cell therapy. At the same time, when the patient is in critical condition, there is not enough waiting time for the preparation of other cells. These problems have brought limitations to the wide application of CAR-T technology. Therefore, an important research direction of CAR-T cell therapy is how to use the T cells of a healthy blood donor to prepare a large number of CAR-T cells for clinical use of a patient. The establishment of this technology will provide a method for treating a patient suffering from T-cell lymphoma, and greatly reduce the cost of CAR-T therapy, which can better ensure the quality of uniformly prepared cells, and a patient in need can get CAR-T therapy immediately.

Several documents are cited throughout the text of this specification, and each of them (including any journal articles or abstracts, published or unpublished patent applications, granted patents, manufacturer's instructions, instructions for use, etc.) is incorporated herein by reference. However, there is no admission that the documents cited herein are in fact prior art to the present invention.

### SUMMARY OF THE INVENTION

The present invention relates to a novel method for preparing a universal CAR-T cell targeting T-cell lymphoma cells, the universal CAR-T cell prepared by the method, and a biological product comprising the universal CAR-T cell. The universal CAR-T cell targeting T-cell lymphoma cells prepared by the preparation method of the present application has no expression of natural TCR, which greatly reduces the graft-versus-host reaction. At the same time, due to the knockout of the B2M gene in the T cell, the expression of class I HLA proteins is also greatly reduced and eliminated, and the autoimmunity of the T cell is reduced. The universal CAR-T cell prepared by the method of the present application can sustainably and efficiently kill T-cell lymphoma cells, and at the same time, the graft-versus-host reaction is greatly reduced, which increases the safety of use.

Therefore, in one aspect, the present invention discloses for the first time a method for preparing a universal CAR-T cell targeting T-cell lymphoma cells by using TCRα/β as a target. Particularly, this application provides the following items:
1. A method for preparing a universal CAR-T cell targeting T-cell lymphoma cells, comprising:
   1) obtaining T cells from a human donor having a healthy lymphatic system;
   2) disrupting the following genomic regions in the T cells by gene editing technology:
      (i) a TRAC genomic region; and
      (ii) a B2M genomic region; and
   3) stably expressing CAR molecules targeting T-cell lymphoma cell TCRα/β in the T cells.
   In some embodiments, the method for preparing a universal CAR-T cell targeting T-cell lymphoma cells according to the present application further comprises:
   4) culturing the cells obtained from step 3) for an appropriate time to deplete T cells retaining expression of natural TCR. The appropriate time is, for example, 3-21, 4-18, 5-16, 6-14, 7-12, 8-10 days to allow cells expressing the CAR to deplete T cells retaining expression of the natural TCR.
2. The method according to item 1, further comprising culturing the cells obtained from step 3) for an appropriate time to deplete T cells retaining expression of the natural TCR.
3. The method according to item 1 or 2, wherein the TRAC genomic region comprises the genomic region from positions 23016448 to 23016490 of human chromosome 14, and the B2M genomic region comprises the genomic region from positions 45003745 to 45003788 of human chromosome 15.
4. The method according to any one of items 1-3, wherein the extracellular region of the CAR molecule targeting T-cell lymphoma cell TCRα/β is an scFv molecule binding to the extracellular region of T-cell lymphoma cell TCRα/β.
5. The method according to item 4, wherein the CAR molecule comprises the following sequences linked by a linker:
   the light chain variable region of the scFv: SEQ ID NO: 19, 
QIVLTQSPAIMSASPGEKVTMTCSATSSVSYMHWYQQKSGTSPKRWIYDTSKLAS
GVPARFSGSGSGTSYSLTISSMEAEDAATYYCQQWSSNPLTFGAGTKLELK;
   the heavy chain variable region of the scFv: SEQ ID NO: 20, 
EVQLQQSGPELVKPGASVKMSCKASGYKFTSYVMHWVKQKPGQGLEWIGYINP
YNDVTKYNEKFKGKATLTSDKSSSTAYMELSSLTSEDSAVHYCARGSYYDYDGFVY
WGQGTLVTVSA.
6. The method according to item 5, wherein the linker is an amino acid sequence comprising (G)n(S)m, and wherein n is a positive integer of 1-20, such as 1, 2, 3, 4, 5, 6, 7, 8, 9, 10, 15, 20; m is a positive integer of 1-10, such as 1, 2, 3, 4, 5, 6, 7, 8, 9, 10.
7. The method according to item 6, wherein the linker sequence is the sequence GGGGSGGGGSGGGGSGGGGS as shown by SEQ ID NO: 21.
8. The method according to any one of items 1-7, wherein the TRAC genomic region and the B2M genomic region are disrupted by homologous recombination, zinc finger nuclease gene editing technology, TALEN gene editing technology, or CRISPR/Cas gene editing technology.
9. The method according to item 8, wherein the gene editing technology is CRISPR/Cas9 gene editing technology.
10. The method according to item 9, wherein:
   (i) the guide RNA (sgRNA) targeting the TRAC genome has a sequence selected from any one of SEQ ID NOs: 2-5; and/or
   (ii) the guide RNA (sgRNA) targeting the B2M genome has a sequence selected from any one of SEQ ID NOs: 6-13.
11. The method according to item 10, wherein the nucleotide sequences encoding the sgRNA and Cas9 are introduced into the T cells concurrently or separately.
12. The method according to item 10 or 11, wherein the sgRNA is chemically modified.
13. The method according to item 12, wherein the chemical modification comprises a 2'-O-methyl modification or an internucleotide 3'-thio modification.
14. The method according to item 13, wherein the chemical modification is 2'-O-methyl modification on each of three bases at the 5' end and three bases at the 3' end of the sgRNA andinternucleotide 3'-thio modification on the three bases at the 5' end and three bases at the 3' end of the sgRNA.
15. The method according to any one of items 12-14, wherein the sgRNA is co-introduced with an mRNA nucleotide sequence encoding Cas9 into the T cell by electroporation.
16. The method according to item 15, wherein the electroporation condition is selected from any one of the following conditions: 150-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; and 250V, 0.5ms.
17. The method according to any one of items 1-16, wherein the TRAC and B2M are individually knocked out with an efficiency of 90%, 95%, 96%, 97%, 98%, 99% or more, and are concurrently knocked out with an efficiency of 75%, 80%, 85%, 86%, 87%, 88%, 89%, 90%, 91%, 92%, 93% or more.
18. The method according to any one of items 1-17, wherein the nucleotide sequence encoding the CAR molecule targeting T-cell lymphoma cell TCRα/β is introduced into the T cells by viral transfection .
19. The method according to item 18, wherein the virus is an adeno-associated virus (AAV) or a lentivirus.
20. The method according to any one of items 1-17, wherein the nucleotide sequence expressing the CAR molecule is introduced into the TRAC gene locus of the T cell by homologous recombination, thereby the nucleotide sequence encoding the CAR molecule is expressed at the TRAC gene locus under the control of a TCR gene promoter.
21. The method according to any one of items 1-20, wherein the cells obtained from step 3) are cultured for 3-21 days to deplete T cells retaining expression of natural TCR.
22. A universal CAR-T cell targeting T-cell lymphoma cells, wherein the CAR-T cell expresses CAR molecules targeting T-cell lymphoma cell TCRα/β.
23. The universal CAR-T cell according to item 22, wherein the CAR molecule comprises the amino acid sequence as shown by SEQ ID NO: 14.
24. The universal CAR-T cell according to item 22 or 23, wherein the CAR-T cell expresses at a low level or does not express one or more proteins selected from the group consisting of: class 1 HLA protein, PD-1, TIM3, and LAG3.
25. A biological product comprising the universal CAR-T cell according to any one of items 22-24.
26. The biological product according to item 25, wherein 98%, 99% or more of the universal CAR-T cells are TCR negative. In some embodiments, 98%, 99% or more of the universal CAR-T cells are negative for TCR and B2M.
27. Use of the universal CAR-T cell according to any one of items 22-24 or the biological product according to item 25 or 26 in the preparation of a medicament for treating T-cell lymphoma.
28. A method for treating T-cell lymphoma, comprising:
   administering a therapeutically effective amount of the universal CAR-T cell according to any one of items 22-24 or the biological product according to item 25 or 26 to a subject suffering from T-cell lymphoma.

### BRIEF DESCRIPTION OF THE DRAWINGS

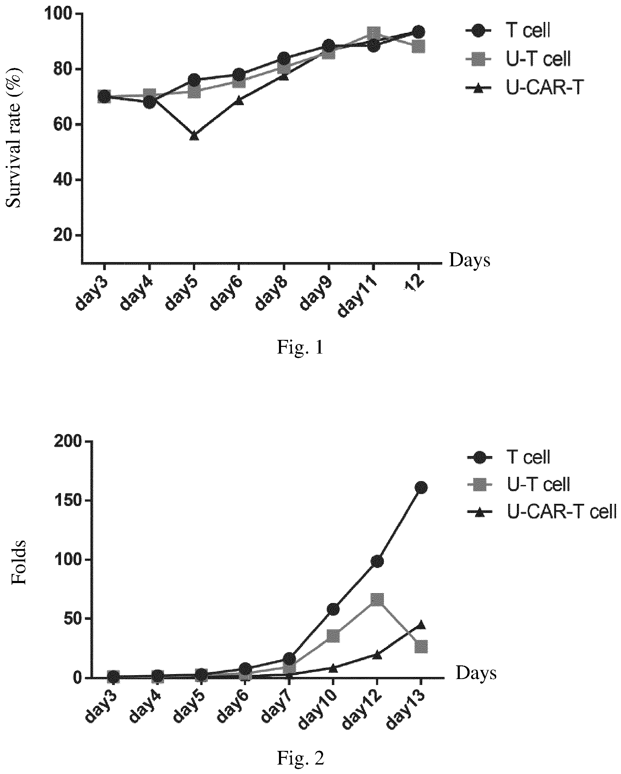

Fig. 1 shows the growth and survival rate of cells after electroporation. The survival rate of T cells and U-CAR-T cells is better, and the cell survival rate reaches more than 90% with the increase of culture time, while at day 5 the survival rate of U-CAR-T cells is lower than the initial survival rate. Since CAR can target TCR, U-CAR-T cells have a "suicide" phenomenon in the early stage, so the survival rate is low, but with the increase of time, the survival rate gradually increases, and the survival rate reaches more than 90% after 9 days of culture.
Fig. 2 shows the growth of the cells after electroporation. The proliferation rate of T cells is the fastest, while those of U-T cells and U-CAR-T cells are slower. After culturing for 12 days, the U-T cells are purified for TCRα/β, and the recovery rate of purification is about 40%. Therefore, the final number of U-CAR-T cells is more than that of U-T cells.

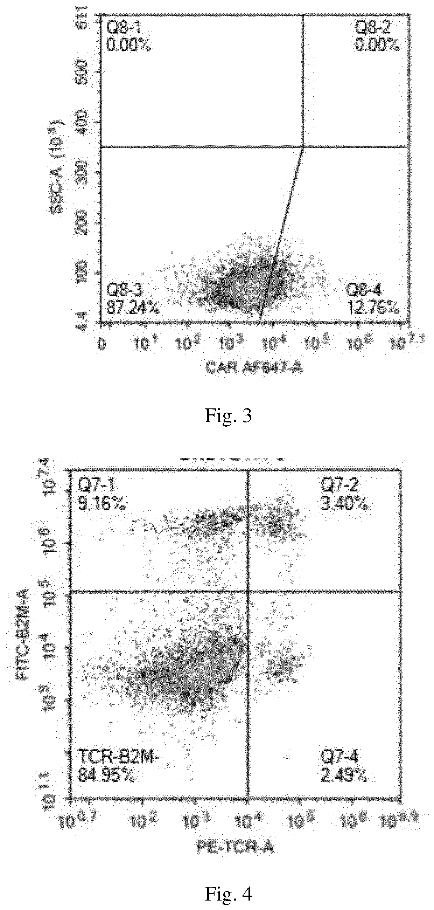

Fig. 3 shows the CAR phenotype analysis of DKO-T cells after culturing by addition with TCRα/β-CAR. It may be seen from the results that, the DKO-T cells after culturing by addition with TCRα/β-CAR have a CAR expression rate of more than 10%.
Fig. 4 shows the result analysis of TRAC/B2M knockout (double knock-out, DKO) in T cells by using optimized sgRNA and CRISPR/Cas9 gene editing technology. The sgRNA is chemically modified, and then the chemically modified sgRNA and Cas9 are delivered into primary T cells through further optimized electroporation conditions for related gene knockout. As shown in the figure, the knockout efficiency of the two genes (i.e., TRAC and B2M) is increased to 84%.

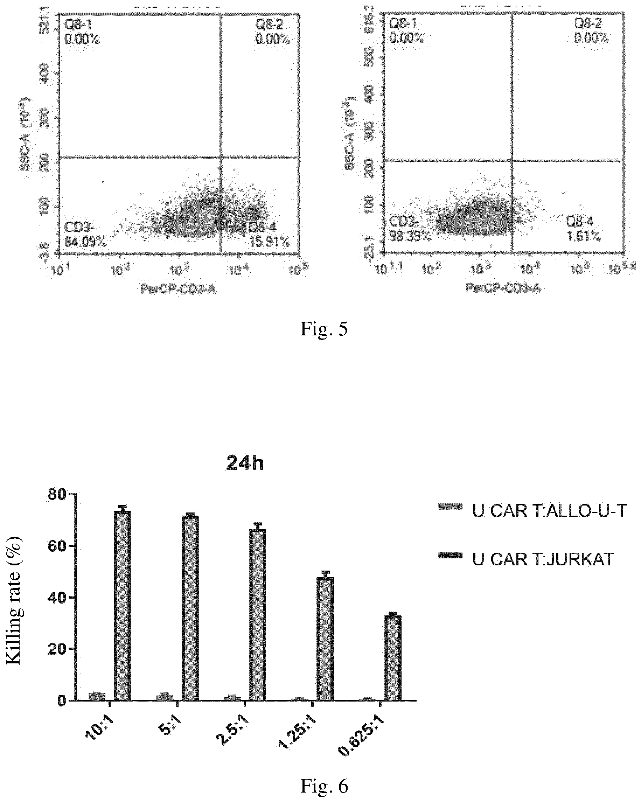

Fig. 5 shows the phenotype analysis of DKO-T cells after culturing by addition with TCRα/β-CAR. It may be seen from the results that, as for the DKO-T cells after culturing by addition with TCRα/β-CAR, the proportion of TCRα/β negative cells is not less than 98%.
Fig. 6 shows the validation and result comparison of the killing function of T cells, DKO-T and DKO CAR-T cells. In this experiment, Jurkat cells are used as target cells; T cells, DKO-T and DKO CAR-T cells are used as effector cells; and in vitro killing experiments are performed with the effector-target ratios of 10:1, 5:1, 2.5:1, 1.25:1, 0.625:1.

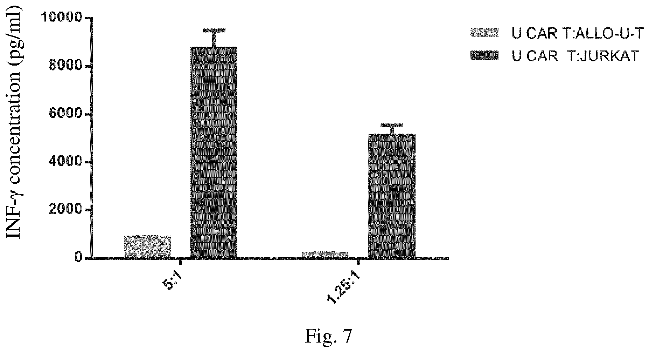

Fig. 7 shows the cytokine release of T cells, DKO-T and DKO CAR-T cells. In this experiment, Jurkat cells are used as target cells; T cells, DKO-T and DKO CAR-T cells are used as effector cells; after in vitro co-cultivation with the effector-target ratios of 10:1, 5:1, 2.5:1, 1.25:1, 0.625:1, the supernatant is taken to detect IFN-γ.

### DETAILED DESCRIPTION OF THE INVENTION

It should be noted that certain terms are used in the description and claims to refer to specific components. It should be understood by those skilled in the art that, the same component may be referred to by different terms. The description and the claims do not use the difference in terms as a way to distinguish the components, but use the difference in function of the components as a criterion for distinguishing. As referred to throughout the specification and claims, "comprising" or "including" is an open-ended term, and should be interpreted as "including but not limited to". Subsequent descriptions in the specification are preferred embodiments for implementing the present invention, however such descriptions are for the purpose of general principles of the specification and are not intended to limit the scope of the present invention. The scope of protection of the present invention shall be defined by the appended claims.

In a first aspect, the present application provides a method for preparing a universal CAR-T cell targeting T-cell lymphoma cells.

In a particular embodiment, provided is a method for preparing a universal CAR-T cell targeting T-cell lymphoma cells, the method comprising:
1) obtaining T cells from a human donor having a healthy lymphatic system;
2) disrupting the following genomic regions in the T cells by gene editing technology:
   (i) a TRAC genomic region; and
   (ii) a B2M genomic region;
3) making CAR molecules targeting T-cell lymphoma cell TCRα/β stable expressed in the T cell; and
4) culturing the cells obtained from step 3).

In the context of this specification, T cells are derived from healthy humans. T cell sources include umbilical cord blood, bone marrow, or peripheral blood mononuclear cells (PBMC). In some embodiments, the T cells are derived from stem cells, such as hematopoietic stem cells at various stages of differentiation. The preparation method described in this application may be used to knock out TRAC, B2M in, for example, PBMC or stem cells and further culture, differentiate, and/or purify corresponding genetically engineered T cells.

The CAR expressed in the CAR-T cells of the present invention comprises a sequentially linked signal peptide, an extracellular binding region, a hinge region, a transmembrane domain and an intracellular signal region. As used herein, the term "signal peptide" refers to a short (e.g., 5-30 amino acids in length) peptide chain that directs the transfer of newly synthesized proteins to the secretory pathway. In the present invention, signal peptides of various proteins in the human body, such as signal peptides of cytokine proteins secreted by the body and leukocyte differentiation antigens (CD molecules) may be used. In some embodiments, the signal peptide is a CD8 signal peptide, e.g., its amino acid sequence is as shown in invention patent application US20140271635A1. In some embodiments, the hinge region may be a hinge region from various antibodies or antigen receptors, particularly a hinge region of CD molecules. In a particular embodiment, the hinge region may be selected from the hinge regions of proteins such as CD8 or CD28. The CD8 or CD28 is a natural marker on the surface of T cells.

In the present invention, the transmembrane domains of various human proteins, especially the transmembrane domains of various different antigen receptors, may be used. The transmembrane domain preferably used is a transmembrane domain of CD molecules. In one embodiment, the transmembrane domain is selected from the transmembrane domains of CD8 proteins.

In the present invention, the hinge region is a CD8a hinge region (CD8-hinge), and its amino acid sequence is shown in the invention patent application US20140271635A1.

The "extracellular binding region" comprises a region specifically recognizing a target antigen. In some embodiments, the extracellular binding region comprises a region specifically recognizing a target tumor cell surface antigen. For example, this region may be an antigen-binding fragment of a scFv or other antibody. As used herein, the term "scFv" refers to a recombinant protein of the variable region of a heavy chain (VH) and the variable region of a light chain (VL) linked by a linker; the linker associates the two domains to ultimately form the antigen-binding site. A scFv is usually an amino acid sequence encoded by a single chain of nucleotides. The above scFv may also include derivatives thereof.

The CAR used in the present invention and each of its domains may be further modified by using conventional techniques known in the art alone or in combination, such as deletion, insertion, substitution, addition, and/or recombination of amino acids, and/or other modification methods. Methods for introducing such modifications into the DNA sequence of an antibody based on its amino acid sequence are well known to those skilled in the art (see, for example, Sambrook, Molecular Cloning: A Laboratory Manual, Cold Spring Harbor Laboratory (1989) N.Y.). The modification is preferably performed at the nucleic acid level.

As used herein, the term "specific recognition" means that the antigen recognition region of the present invention does not cross-react with or substantially does not cross-react with any polypeptide other than the target antigen. The degree of its specificity may be judged by immunological techniques, including but not limited to immunoblotting, immunoaffinity chromatography, flow cytometry, and the like.

The extracellular binding region is an antigen binding region that specifically recognizes TCRα/β.

In the present invention, the intracellular signal region is an intracellular signal region of CD137(4-1BB) protein. The CD3 molecule consists of five subunits, wherein the CD3ζ subunit (also known as CD3 zeta, abbreviated as ζ) contains 3 ITAM motifs, which are important signal transduction regions in the TCR-CD3 complex. FcεRIγ is mainly distributed on the surface of mast cells and basophils, and it contains an ITAM motif similar to CD3ζ in structure, distribution and function. In addition, as mentioned above, CD137 is a costimulatory signaling molecule, and the co-stimulatory effect produced by its intracellular signaling segment after binding to the respective ligands causes the continuous proliferation of T cells and can increase the secretion of IL-2 and IFN- γ and other cytokines, while improving the *in vivo* survival cycle and anti-tumor effect of CAR-T cells. In certain embodiments, the signal generated by CAR alone is not sufficient to fully activate natural T cells, and a sequence for TCR initiation antigen-dependent primary activation (primary intracellular signaling region) and a sequence acting in antigen-independent manner to provide a costimulatory signal (costimulatory domain). Primary signaling domains regulate primary activation of the TCR complex in a stimulatory or inhibitory manner. Primary intracellular signaling regions acting in a stimulatory manner may contain signaling motifs, known as immunoreceptor tyrosine activation motifs (ITAMs). The primary cytoplasmic signaling sequence containing ITAM in the present invention is CD3ζ. In one embodiment, the primary signaling domain comprises a modified ITAM domain, e.g., a mutated ITAM domain whose activity is altered (e.g., increased or decreased) as compared to the natural ITAM domain; or the primary intracellular signaling region comprises a truncated ITAM. In one embodiment, the primary signaling domain comprises one or more ITAM motifs.

A costimulatory signaling domain refers to a portion of the TCR that comprises the intracellular domain of a costimulatory molecule. Costimulatory molecules are cell surface molecules other than antigen receptors or their ligands that are required for lymphocytes to efficiently response to antigens. The costimulatory molecule region of the present invention is 4-1BB (CD137).

The "T cell receptor (TCR)" is a characteristic marker on the surface of all T cells, and it binds to CD3 to form a TCR-CD3 complex. TCR consists of two peptide chains, α and β, wherein each peptide chain may be divided into a variable region (V region), a constant region (C region), a transmembrane domain and a cytoplasmic region. The TCR molecule belongs to the immunoglobulin superfamily, and its antigen specificity exists in the V region; and the V region (Vα, Vβ) has three hypervariable regions (CDR1, CDR2, and CDR3); wherein CDR3 has the largest variation which directly determines the antigen binding specificity of TCR. When TCR recognizes the MHC-antigen peptide complex, CDR1 and CDR2 recognize and bind to the side wall of the antigen-binding groove of the MHC molecule, while CDR3 directly binds to the antigen peptide. TCRs are divided into two categories: TCR1 and TCR2; wherein TCR1 consists of two chains, γ and δ; and TCR2 consists of two chains, α and β. In peripheral blood, 90%-95% of T cells express TCR2; and any T cell expresses only one of TCR2 and TCR1.

"β2 microglobulin (B2M)" is the β chain (light chain) part of the human leukocyte antigen (HLA) on cell surface, and is a single-chain polypeptide of 99 amino acids with a molecular mass of 11800.

As used herein, "Indel" stands for insertion/deletion, i.e., insertion and deletion mutations.

"Graft-versus-host reaction (GVHD)" refers to a reaction caused by the presence of immunogenetic differences between donor and recipient, e.g., on the one hand, when donor cells, such as T lymphocytes of an immunocompetent donor, enter the body of the recipient (patient) and proliferate to a certain extent, the normal cells or tissues of the recipient (patient) are mistaken for the target to attack by the donor cells; on the other hand, the donor cells as allogeneic cells, the recipient's normal immune system may also clear them to produce a "host-versus-graft reaction (HVGR)".

HVGR and GVHR-related genes include TCR and HLA molecule-related genes. T lymphocytes which are knocked out both the above two genes at the same time will not cause graft-versus-host disease (GVHD) when they are infused back into an allogeneic patient, so they may be called "universal T cells". For example, the two genes of a single TRAC gene encoding TCRα chain and a single TRBC gene encoding TCRβ form a complete and functional TCR complex. TRAC knockout can cause TCR inactivation, while B2M is an MHCI-related gene. T lymphocytes which are knocked out both the above two genes at the same time will not cause graft-versus-host disease (GVHD) when they are infused back into an allogeneic patient.

"CAR-T" is an abbreviation for "chimeric antigen receptor T cells", wherein the chimeric antigen receptor (CAR) is the core component of CAR-T, which enables T cells to recognize target cell (e.g. tumor) antigens in an HLA-independent manner, thereby allowing CAR-engineered T cells to recognize a wider range of targets, as compared with the natural T-cell surface receptor TCR. In some embodiments, the tumor-targeted CAR is designed to include a tumor-associated antigen (TAA) binding region (e.g., a scFV segment typically derived from the antigen binding region of a monoclonal antibody), an extracellular hinge region, a transmembrane domain and an intracellular signaling region. The choice of target antigen is a key determinant for the specificity and efficacy of CAR, and safety of the genetically modified T cells themselves.

"Universal CAR-T cells" refer to CAR-T cells that can target specific target cell (such as tumor)-related markers and whose TCR and MHC functions on the cell surface are inactivated, thereby reducing the immune rejection caused by allogeneic cell therapy.

CAR-T treatment of autologous cells requires blood extraction to separate the patient's own T lymphocytes for preparing the CAR-T. On the one hand, since the T cells of a patient are cancerous, it is impossible to use the patient's own T cells for preparing the CAR-T. Alternatively, during the process of preparing the CAR-T, if an emergency occurs and the prepared cells cannot be infused back into the patient in time, the therapeutic effect will also be affected; thus the universal CAR-T cells that may be used for allogeneic therapy have great advantages in the above situations.

Adoptive cellular therapy (ACT), adoptive immunotherapy, for example, tumor adoptive immunotherapy is such a therapy, it refers to the treatment of immune cells *in vitro,* such as adding specific antigens, modifying the molecules expressed by immune cells, or stimulating the immune cells with cytokines etc., screening and massively proliferating immune effector cells which kill target cells (such as tumors) with high specificity, and then infusing them into a patient to kill target cells (such as tumors); and this is a passive immunotherapy.

"Stable expression" means continuous expression. The stable expression of a transgene in a host cell may be achieved by stable transfection, thereby integrating a target gene into the chromosomal DNA of the cell and instructing the synthesis of an appropriate amount of the target protein. In the case of stable transfection (e.g., by viral transfection), an exogenous gene may be integrated into the genome of the cell, then the exogenous gene becomes part of the cell genome and thus replicated, and the daughter cells of the stably transfected cell also express the exogenous gene, thereby achieving stable expression.

In another particular embodiment, the TRAC genomic region comprises the genomic region from positions 23016448 to 23016490 of human chromosome 14, and the B2M genomic region comprises the genomic region from positions 45003745 to 45003788 of human chromosome 15.

In the context of this specification, "genome" conforms to the general definition in the field of molecular biology, i.e., it refers to the sum of all genetic material of an organism. This genetic material may include DNA or RNA.

In yet another particular embodiment, the extracellular region of the CAR molecule which targets T-cell lymphoma cell TCRα/β is a scFv molecule that binds to the extracellular region of T-cell lymphoma cell TCRα/β.

In the context of this specification, "scFv" refers to a single chain antibody (single chain antibody fragment), which is an antibody formed by linking a heavy chain variable region and a light chain variable region with a short peptide (linker) of 15-20 amino acids. Therefore, the scFv molecule may be understood as the extracellular part of the CAR molecule, and it consists of the light chain variable region, the linker, and the heavy chain variable region.

In a particular embodiment, the CAR molecule comprises the following sequences linked by a linker:
light chain variable region of scFv, which is as shown by SEQ ID NO: 19 
QIVLTQSPAIMSASPGEKVTMTCSATSSVSYMHWYQQKSGTSPKRWIYDTSKLAS
GVPARFSGSGSGTSYSLTISSMEAEDAATYYCQQWSSNPLTFGAGTKLELK;
 heavy chain variable region of scFv, which is as shown by SEQ ID NO: 20

EVQLQQSGPELVKPGASVKMSCKASGYKFTSYVMHWVKQKPGQGLEWIGYINP
YNDVTKYNEKFKGKATLTSDKSSSTAYMELSSLTSEDSAVHYCARGSYYDYDGFVY
WGQGTLVTVSA.

In yet another particular embodiment, the linker is an amino acid sequence comprising (G)n(S)m, wherein n is a positive integer of 1-20, and m is a positive integer of 1-10. Particularly, n may be 1, 2, 3, 4, 5, 6, 7, 8, 9, 10, 11, 12, 13, 14, 15, 16, 17, 18, 19, or 20; and m may be 1, 2, 3, 4, 5, 6, 7, 8, 9, or 10. Herein, G is the abbreviated form of glycine, which can also be abbreviated as Gly; S is the abbreviated form of serine, which can also be abbreviated as Ser.

In a particular embodiment, the linker sequence is the sequence GGGGSGGGGSGGGGSGGGGS which is as shown by SEQ ID NO: 21.

In yet another particular embodiment, the amino acid sequence of the CAR molecule comprises or consists of the amino acid sequence as shown by SEQ ID NO: 14.

In a particular embodiment, the TRAC genomic region and the B2M genomic region are disrupted by homologous recombination, zinc finger nuclease gene editing technology, TALEN gene editing technology, or CRISPR/Cas gene editing technology.

In yet another particular embodiment, the gene editing technology is CRISPR/Cas9 technology.

As used herein, "CRISPR/Cas" is a gene editing technology that includes, but is not limited to, various naturally occurring or artificially designed CRISPR/Cas systems, such as the CRISPR/Cas9 system. The naturally occurring CRISPR/Cas system is an adaptive immune defense of bacteria and archaea which is formed during a long-term evolution, and it may be used to fight invading viruses and foreign DNA. For example, CRISPR/Cas9 works by combining crRNA (CRISPR-derived RNA) with tracrRNA (trans-activating RNA) through base pairing to form a tracrRNA/crRNA complex, and this complex guides the nuclease Cas9 protein to cleave double-stranded DNA at a target site in the sequence which is paired with crRNA. By artificially designing tracrRNA and crRNA, sgRNA (single guide RNA) with guiding function may be formed by modification, and it is sufficient to guide a site-directed cleavage of DNA by Cas9. As an RNA-guided dsDNA-binding protein, the Cas9 effector nuclease can co-localize RNA, DNA, and protein, thereby possessing great engineering potential. CRISPR/Cas systems can use class I, class II, or class III Cas proteins. In some embodiments of the invention, the method uses Cas9. Other suitable CRISPR/Cas systems include, but are not limited to, those systems and methods described in WO2013176772, WO2014065596, WO2014018423, and US 8,697,359.

In yet another particular embodiment, the following guide RNAs are used in the CRISPR/Cas9 method of the present application:
(i) a guide RNA (sgRNA) targeting the TRAC genome, which has a sequence selected from any one of SEQ ID NOs: 2-5; and
(ii) a guide RNA (sgRNA) targeting the B2M genome, which has a sequence selected from any one of SEQ ID NOs: 6-13.

In the present invention, "sgRNA (single guide RNA)" and "gRNA (guide RNA)" may be "single guide RNA", "synthetic guide RNA", or "guide RNA", and they may be used interchangeably. The sgRNA of the present invention comprises a guide sequence targeting the target sequence.

In general, a guide sequence in a sgRNA is any polynucleotide sequence that has sufficient complementarity to the target polynucleotide sequence so as to hybridize to the target sequence and direct the sequence-specific binding of the CRISPR complex to the target sequence. In some embodiments, when optimal alignment is performed by using an appropriate alignment algorithm, the degree of complementarity between the guide sequence and its corresponding target sequence is about or greater than about 80%, 85%, 90%, 95%, 97.5%, 99%, or more. Optimal alignment may be determined by using any suitable algorithm for aligning sequences, and the non-limiting examples include Smith-Waterman algorithm, Needleman-Wimsch algorithm, Burrows-Wheeler Transform based algorithms (e.g. Burrows Wheeler Aligner), ClustalW, Clutai X, BLAT, Novoalign (Novocraft Technologies, ELAND (Illumina, San Diego, CA), SOAP (available at soap.genomics.org.cn) and Maq (available at maq.sourceforge.net). In some embodiments, the length of a guide sequence may be about or greater than about 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, or more nucleotides. In some embodiments, the length of a guide sequence is less than about 75, 70, 65, 60, 55, 50, 45, 40, 35, 30, 25, 20, 15, 12, or fewer nucleotides. The ability of a guide sequence to direct sequence-specific binding of the CRISPR complex to the target sequence may be assessed by any suitable assay. For example, a host cell with a corresponding target sequence may be provided with components of a CRISPR system (including the guide sequence to be tested) sufficient to form a CRISPR complex; for example, it may be performed by transfecting with a vector encoding the CRISPR sequence component, then assessing the preferable cleavage within the target sequence. Likewise, cleavage of a target polynucleotide sequence may be evaluated in a test tube by providing the target sequence, components of a CRISPR complex (including a guide sequence to be tested, and a control guide sequence different from the guide sequence), and comparing the rate of binding or cleavage at the target sequence for the test and control guide sequences. The above assays and assessments may also be performed by using other methods known to those skilled in the art.

In yet another particular embodiment, the nucleotide sequences encoding the sgRNA and Cas9 are introduced into the T cells concurrently or separately.

In a particular embodiment, the sgRNA and the mRNA nucleotide sequence encoding Cas9 are co-introduced into the T cells by electroporation.

Particularly, the TRAC-targeting sgRNA, B2M-targeting sgRNA and/or Cas9-encoding nucleotides (e.g., mRNA) are introduced into the T cells by electroporation. In some embodiments, the TRAC-targeting sgRNA, B2M-targeting sgRNA, and Cas9-encoding nucleotides are co-introduced into T cells by electroporation.

Particularly, the Cas9-encoding nucleotides are mRNAs, such as mRNAs comprising an ARCA cap. In some embodiments, the Cas9-encoding nucleotides are in a viral vector, such as a lentiviral vector. In some embodiments, the Cas9-encoding nucleotides comprise the sequence as shown by SEQ ID NO: 1. In some embodiments, the TRAC-targeting sgRNA, the B2M-targeting sgRNA and the Cas9-encoding nucleotides are in the same vector.

Particularly, the electroporation condition is selected from any one of the following conditions: 150-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; and 250V, 0.5ms.

In the present application, the TRAC-targeting sgRNA and the B2M-targeting sgRNA are concurrently introduced into T cells. Particularly, when the TRAC-targeting sgRNA and the B2M-targeting sgRNA are concurrently introduced into T cells, the amounts of the TRAC-targeting sgRNA and the B2M-targeting sgRNA may be similar or equivalent. In some embodiments, the TRAC-targeting sgRNA, the B2M-targeting sgRNA are introduced into the T cells one by one in any suitable order. In some embodiments, the TRAC-targeting sgRNA, the B2M-targeting sgRNA and the Cas9-encoding nucleotides are introduced into T cells concurrently. In some embodiments, the Cas9-encoding nucleotides are introduced into T cells prior to the TRAC-targeting sgRNA and the B2M-targeting sgRNA. Particularly, T cells comprise Cas9-encoding nucleotides or Cas9 proteins.

In the present application, the alpha chain constant coding region (i.e., TRAC) gene of the TCR is knocked out. For example, in some particular embodiments, the TCRα chain constant coding region gene of the present invention is introduced into one of the TRAC-sg 2, 3, 4, and 6 molecules of the cell (see **Table 1** for details).

**Table 1**

| | | |
|---|---|---|
| T2 | GCTGGTACACGGCAGGGTCA | SEQ ID NO: 2 |
| T3 | CTCTCAGCTGGTACACGGCA | SEQ ID NO: 3 |
| T4 | ATTTGTTTGAGAATCAAAAT | SEQ ID NO: 4 |
| T6 | TCTCTCAGCTGGTACACGGC | SEQ ID NO: 5 |
| B1 | ACTCTCTCTTTCTGGCCTGG | SEQ ID NO: 6 |
| B2 | GAGTAGCGCGAGCACAGCTA | SEQ ID NO: 7 |
| B3 | CGCGAGCACAGCTAAGGCCA | SEQ ID NO: 8 |
| B4 | TCACGTCATCCAGCAGAGAA | SEQ ID NO: 9 |
| B5 | GCTACTCTCTCTTTCTGGCC | SEQ ID NO: 10 |
| B6 | TTTGACTTTCCATTCTCTGC | SEQ ID NO: 11 |
| B7 | CGTGAGTAAACCTGAATCTT | SEQ ID NO: 12 |
| B8 | CTCGCGCTACTCTCTCTTTC | SEQ ID NO: 13 |

In a particular embodiment, the sgRNA is chemically modified.

In yet another particular embodiment, the chemical modification comprises a 2'-O-methyl modification or an internucleotide 3'-thio modification.

In yet another particular embodiment, the chemical modification is 2'-O-methyl modification on each of three bases at the 5' end and three bases at the 3' end of the sgRNA and internucleotide 3'-thio modification on the three bases at the 5' end and three bases at the 3' end of the sgRNA.

In addition to the specific chemical modifications used in the examples, other modification methods are also included, for example, the chemical modification methods reported in Deleavey GF1, Damha MJ. Designing chemically modified oligonucleotides for targeted gene silencing. Chem Biol. 2012 Aug 24; 19(8): 937-54; and Hendel et al., Chemically modified guide RNAs enhance CRISPR-Cas genome editing in human primary cells. Nat Biotechnol. 2015 Sep;33(9):985-989.

In the present invention, the chemically modified sgRNA and Cas9-encoding gene are co-introduced into T cells by electroporation, resulting in efficient gene editing efficiency (for example, expressed in TCRα/β-/B2M-%), wherein the chemical modification of sgRNA is one of the key factors in the present invention. The data in the examples show that, if the chemically unmodified sgRNA is co-introduced with the Cas9 mRNA by electroporation, the Indels efficiency is much lower than the gene editing efficiency obtained when the chemically modified sgRNA is co-introduced with the Cas9 mRNA by electroporation. In a particular embodiment, the TRAC and B2M are individually knocked out with an efficiency of 90%, 92%, 94%, 96%, 98%, or more, and concurrently knocked out with an efficiency of 75%, 80%, 85%, or more.

"Knockout efficiency" may be expressed at gene level as the efficiency of producing a gene knockout INDEL, or it may be expressed at cellular level as the percentage of cells in which the gene knockout causes protein expression of the gene to disappear or significantly decrease. In the present invention, "knockout efficiency" refers to the knockout efficiency calculated based on the latter. Those skilled in the art can understand that, high knockout efficiency can improve the harvest rate of target cells and reduce production costs and treatment costs.

Particularly, gene-edited T cells (e.g., universal T cells) are further screened to obtain higher-purity dual-gene (TRAC and B2M) knockout T cells. For example, gene-edited T cells with low TRAC and B2M expression (e.g., universal T cells) may be screened by FACS.

Particularly, the TCR and/or HLA genes of the universal T cells of the present invention are knocked out. In some particular embodiments, the alpha chain constant coding region (i.e., TRAC) gene of the TCR is knocked out. The coding region of B2M is knocked out.

In a particular embodiment, the nucleotide sequence encoding the CAR molecule which targets T-cell lymphoma cell TCRα/β is introduced into the T cell by viral transfection for stable expression. Particularly, the virus is an adeno-associated virus (AAV) or a lentivirus.

In some particular embodiments, T cells expressing natural TCR are eliminated following the culture process of step 4). In this application, this clearing process is also referred to as "purification" and is an alternative to the traditional method of purifying U (universal) CAR-T cells by using magnetic beads. Purification by this method requires no additional purification steps, has no loss of cells, and also reduces time and cost.

In a second aspect, the present application relates to a universal CAR-T cell targeting T-cell lymphoma cells.

In a particular embodiment, provided is a universal CAR-T cell targeting T-cell lymphoma cells, and the cell expresses CAR molecules targeting T-cell lymphoma cell TCRα/β, without natural TCR molecules. In yet another particular embodiment, the CAR molecule comprises the amino acid sequence as shown by SEQ ID NO: 14.

In yet another particular embodiment, provided is the above universal CAR-T cell, which expresses at a low level or does not express one or more proteins selected from the group consisting of: HLA class 1 protein, PD-1, TIM3, and LAG3.

In the context of this specification, PD-1 (programmed death receptor 1), also known as CD279 (cluster of differentiation 279), is an important immunosuppressive molecule. The immune system is regulated by down-regulating the immune system's response to human cells, as well as by suppressing inflammatory activity of T cells, thereby promoting self-tolerance. By knocking out the PD-1, TIM3, and/or LAG3 genes, these proteins may be expressed at a low level, or not be expressed in T cells, thereby reducing the subject's tolerance to infused foreign T cells, reducing their clearance, and thus extending the time that CAR-T cells survive and function in the subject.

In a particular embodiment, provided is a biological product which comprises the above universal CAR-T cells.

In a third aspect, the present application provides use of a universal CAR-T cell.

In a particular embodiment, provided is use of the above universal CAR-T cell or biological product in the preparation of a medicament for treating T-cell lymphoma.

In the context of this specification, "T-cell lymphomas (TCL)" are malignant tumors that appear due to abnormal T-cell proliferation; they belong to a special type of non-Hodgkin's lymphoma, with a low incidence but a high degree of malignancy, and the one-year survival rate of patients is low. The clinical manifestations of this disease are lymphadenopathy, low resistance, hypercalcemia, bone erosion and the like. T-cell lymphomas are broadly divided into two categories: pre-T-cell tumors and post-thymic T-cell lymphomas, which can originate in lymph nodes, extranodal tissue, or the skin. Particular examples are anaplastic large cell lymphoma (ALCL) and peripheral T-cell lymphoma (PTCL). Mature or peripheral T-cell lymphomas have a poorer prognosis, as compared with aggressive B-cell lymphomas. Peripheral T-cell lymphomas originating from the innate immune system tend to occur in adolescents, mostly with extranodal involvement, and the predilection sites are the skin and mucous membranes, while peripheral T-cell lymphomas originating from other immune systems tend to occur in adults, mostly with lymph node involvement, accounting for more than 2/3 of PTCL.

In yet another particular embodiment, the above use comprises: administering a therapeutically effective amount of the above universal CAR-T cells or biological products to a subject suffering from T-cell lymphoma.

The CAR-T cells involved in the present invention may be administered to a subject in need thereof by a route conventionally used in the administration of a pharmaceutical product comprising cellular components, such as an intravenous infusion route. The dose to be administered may be specifically determined based on the condition and general health of the subject.

A source of T cells is obtained from the subject prior to expansion and genetic modification. The term "subject" is intended to include any living organisms (e.g., mammals) capable of eliciting an immune response. Examples of subjects include human. T cells may be obtained from a variety of sources, including peripheral blood mononuclear cells, bone marrow, lymph node tissue, cord blood, thymus tissue, tissue from infection sites, ascites, pleural effusion, spleen tissue, and tumors (except T-cell lymphoma). The T cells of the present invention can also be derived from hematopoietic stem cells at various stages of differentiation. In directed differentiation culture conditions, hematopoietic stem cells differentiate into T cells. In certain aspects of the invention, various T cell lines available in the art may be used.

In certain aspects of the invention, T cells may be obtained from blood collected from a subject by using a variety of techniques known to the skilled artisan, such as Ficoll^{™} separation. Cells can also be obtained from an individual's circulating blood by apheresis. Apheresis products typically contain lymphocytes, including T cells, monocytes, granulocytes, B cells, other nucleated leukocytes, red blood cells, and platelets. In one aspect, cells collected by apheresis may be washed to remove the plasma fraction, then placing them in an appropriate buffer or medium for subsequent processing steps.

T cells may be isolated from peripheral blood lymphocytes by lysing red blood cells and depleting monocytes by panning, e.g., by PERCOLL^{™} gradient centrifugation or countercurrent centrifugation. Specific T cell subsets such as CD3+, CD28+, CD4+, CD8+, CD45RA+, CCR7+, CD62L+, and CD45RO+ T cells may be further isolated by positive or negative selection techniques. For example, in one aspect, T cells are separated by incubating with anti-CD3/anti-CD28 (e.g., 3x28) conjugated beads, such as DYNABEADS^{™} M-450CD3/CD28T, for a time sufficient to positively select the desired T cells. Tumor infiltrating lymphocytes (TILs) may be isolated from the tumor tissue.

The content of the present invention will be described below through particular examples. It should be understood that, the particular examples are only for illustrative purposes, and do not mean that the content of the present invention is limited to the particular examples. Several documents are cited throughout the text of this specification, and each of them (including any journal articles or abstracts, published or unpublished patent applications, granted patents, manufacturer's instructions, instructions for use, etc.) is incorporated herein by reference. However, there is no admission that the documents cited herein are in fact prior art to the present invention.

### EXAMPLES

### Example 1: Preparation of Universal T Cells

### Preparation and Expansion of Universal CAR-T Cells

T cells are obtained by sorting from human peripheral blood, and the sorted T cells are activated with cytokines, and then the cell density is adjusted to 1×10⁶ cells/mL with medium for culturing T cells. After 72h, the state of the cells is observed, and the cell suspension is collected, centrifuging at 300 g for 7 min, discarding the supernatant, washing twice with DPBS solution (manufacturer: Gibco; catalogue number: 1924294); and then the cell density is adjusted to 2.5×10⁷ cells/mL. Synthetic Cas9 mRNA and synthetic sgRNA are used to mix T cells and RNA to a final concentration of 2.5×10⁶ cells and 2µg RNA (2µg for each of Cas9 mRNA and sgRNA) per 100µL, and then electroporator BTX Agile pulse MAX is used to introduce the RNA into the cells for culturing. The growth status of the cells is observed every day, and the cells are counted every other day and supplemented with liquid. On day 4, lentivirus packaged with CAR (see below for the specific sequence) (anti-TCRα/βscFv, linker, CD8 alpha hinge region, CD8 transmembrane domain, 4-11BB signaling domain, 4-11BB signaling domain and CD3 zeta, the specific structures are shown in US20140271635A1) is added according to the ratio of MOI=2-10.

The detection results of the survival rate of the cells during the expansion culture process are shown in Fig. 1. In the early stage of cell culture, around day 4-8, the survival rate of U-CAR-T (universal T cells introduced with CAR targeting TCRα/β) is poor, since the cells may kill themselves and retain expression of natural TCRα/β of T cells, but the survival rate of cells in the later stage is not significantly different from the other two groups. Fig. 2 shows the folds of the expansion of cells after 13 days of culture. Due to the "suicide" of CAR-added cells, the expansion fold is not as good as that of other control groups. However, U-T requires further purification, resulting in a lower number of harvested cells than the universal CAR-T that does not require purification.

In the process of cell expansion and culture, flow cytometry is used to monitor the expression ratio of CAR. The results are respectively shown in Fig. 3, and the positive rate of CAR is about 12.76%. The chemically modified RNA is used for gene knockout, and its editing efficiency is stable and efficient; as shown in Fig. 4, the TCR and B2M cells are tested by flow cytometry, and the efficiency of double gene knockout is 84.95%. The double-gene knockout T cells with CAR targeting TCRα/β are added, as shown in Fig. 5, the negative rate of TCR and B2M is more than 98%, the cells expressing TCR and B2M are further eliminated, thereby bringing the effect of further purification, simplifying the process, saving time and cost, and obtaining more cells at the same time.

### Screening of Universal U-T Cells

TCR and B2M negative, CD4 and CD8 positive T cells are screened according to the following method.

Immunomagnetic bead technology is used to screen out TCR and B2M negative, and CD4 and CD8 positive T cells, and the status of edited T cells is monitored by survival rate of the T cells. The specific steps are as follows:
Step 1: on day 12-14, the T cells after electroporation are collected, centrifuging at 400G for 5 min; the supernatant is discarded, Easy buffer is added to the cells to adjust to 1×10⁸/ml, and then the cells are transferred to a 5ml test tube for flow cytometry; the screening magnetic beads are used to remove the cells retaining expression of TCR and B2M in the T cells, the final products obtained by this screening are universal T cells.
Step 2: a small amount of T cells are taken to conduct flow cytometry, concurrently staining the biomarkers of TCR and B2M on cell surface. If the positive rate of TCR and/or B2M is less than 1%, the next step may be performed. In this example, the positive rate of TCR is 1%, and the positive rate of TCR and B2M DKO in T cells is <0.79%.

### Example 2: Functional Verification of the Universal CAR-T Obtained in Example 1

The killing effect of the universal CAR-T cells (i.e., effector cells) obtained in Example 1 on T-cell acute lymphoblastic leukemia cells is observed.

### A. In Vitro Killing Effect on Specific Tumor Cells

The experimental steps of the present invention are as follows:

### Step 1: Target Cell Labeling

Target cells (human lymphoma cells Jurkat, all cells from ATCC) are labeled by using the CELL TRACE^{™} Far Red Cell Proliferation Kit (manufacturer: Gibco; Cat. No. 1888569).
1. Cell Trace^{™} Far Red Cell Proliferation is diluted to 1mmol solution with double distilled water;
2. 1×10⁶ target cells are taken to centrifuge at 400g for 5 min, then removing the supernatant;
3. 1 µl of Cell Trace^{™} Far Red Cell Proliferation solution is added to incubate at 37°C for 20 min in the dark;
4. The cells are added to culture medium for T cells to incubate at 37°C for 5 min.
5. After centrifugation at 400g for 5 min, the supernatant is removed, and the labeling is completed.

### Step 2: Detecting the Killing of Target Cells by Effector Cells

The labeled target cells are resuspended in R1640+10% FBS medium at a density of 2×10⁵ cells/mL, and 500 µL of the cells is taken to add to a 48-well plate. According to an appropriate effector-target ratio (2.5:1, 1.25:1, 0.6:1), 500 µL of effector cells are added to each well, while T cells and healthy human cord blood CAR-T cells (on day 2 of T cell culture, CAR-T prepared by adding lentivirus packaged with CAR (see US20140271635A1 for the specific structure) at a ratio of MOI=2-10) are used as control cells, with 3 parallel samples in each group, and a separate target cell group is designed to detect its mortality; incubating at 37°C, 5% CO₂ for 12-16 h, then centrifuging at 400 g for 5 min, the cell pellet is taken to resuspend in 150 µl of DPBS (manufacturer: Gibco; catalogue number: 1924294). After staining with PI (manufacturer: Sigma; catalogue number: P4170), the mortality rate of target cells is detected by flow cytometry, and the results are shown in Fig. 6, the universal CAR-T cells show efficient and specific killing effect; at the same time, it has almost no killing function to non-specific target cells.

### Step 3: ELISA Assay of Cytokine Release

The labeled target cells are resuspended in RPMI 1640+10% FBS at a density of 2×10⁵ cells/mL, and 500 µL of the cells is taken to add to a 48-well plate. According to an appropriate effector-target ratio (10:1), 500 µL of effector cells are added to each well, while T cells and healthy human cord blood CAR-T cells are used as control cells, with 3 parallel samples in each group, and a separate target cell group is designed; incubating at 37°C, 5% CO₂ for 12-16 h, then 100µL of cultural supernatant to centrifuge at 400 g for 5 min, discarding the precipitation; the supernatant is taken to use LEGEND MAXTM Human IL-2/IFN-gama (manufacturer: Biolegend; catalogue numbers: 431807, 430108 respectively) ) kit to detect the factor release according to the instructions for use; and the results are shown in Fig. 7.

Although the embodiments of the present invention have been described above with reference to the accompanying drawings, the present invention is not limited to the above particular embodiments and application fields, and the above particular embodiments are only illustrative and instructive, rather than restrictive. Those of ordinary skill in the art can also make many modified forms under the inspiration of this specification and without departing from the scope of protection of the claims of the present invention, which all belong to the protection scope of the present invention.

### Sequence Listing

SEQ ID NO: 1: Cas9 mRNA sequence 
gacaagaaguacagcaucggccuggacaucggcaccaacucugugggcugggccgugaucaccgacgaguacaaggugcc
cagcaagaaauucaaggugcugggcaacaccgaccggcacagcaucaagaagaaccugaucggagcccugcuguucgaca
gcggcgaaacagccgaggccacccggcugaagagaaccgccagaagaagauacaccagacggaagaaccggaucugcuauc
ugcaagagaucuucagcaacgagauggccaagguggacgacagcuucuuccacagacuggaagaguccuuccugguggaa
gaggauaagaagcacgagcggcaccccaucuucggcaacaucguggacgagguggccuaccacgagaaguaccccaccauc
uaccaccugagaaagaaacugguggacagcaccgacaaggccgaccugcggcugaucuaucuggcccuggcccacaugau
caaguuccggggccacuuccugaucgagggcgaccugaaccccgacaacagcgacguggacaagcuguucauccagcugg
ugcagaccuacaaccagcuguucgaggaaaaccccaucaacgccagcggcguggacgccaaggccauccugucugccagac
ugagcaagagcagacggcuggaaaaucugaucgcccagcugcccggcgagaagaagaauggccuguucggcaaccugauu
gcccugagccugggccugacccccaacuucaagagcaacuucgaccuggccgaggaugccaaacugcagcugagcaagga
caccuacgacgacgaccuggacaaccugcuggcccagaucggcgaccaguacgccgaccuguuucuggccgccaagaaccu
guccgacgccauccugcugagcgacauccugagagugaacaccgagaucaccaaggcccccugagcgccucuaugaucaa
gagauacgacgagcaccaccaggaccugacccugcugaaagcucucgugcggcagcagcugccugagaaguacaaagaga
uuuucuucgaccagagcaagaacggcuacgccggcuacauugacggcggagccagccaggaagaguucuacaaguucauc
aagcccauccuggaaaagauggacggcaccgaggaacugcucgugaagcugaacagagaggaccugcugcggaagcagcg
gaccuucgacaacggcagcauccccaccagauccaccugggagagcugcacgccauucugcggcggcaggaagauuuuu
 
acccauuccugaaggacaaccgggaaaagaucgagaagauccugaccuuccgcauccccuacuacgugggcccucuggcca
ggggaaacagcagauucgccuggaugaccagaaagagcgaggaaaccaucacccccuggaacuucgaggaagugguggac
aagggcgcuuccgcccagagcuucaucgagcggaugaccaacuucgauaagaaccugcccaacgagaaggugcugcccaa
gcacagccugcuguacgaguacuucaccguguauaacgagcugaccaaagugaaauacgugaccgagggaaugagaaagc
ccgccuuccugagcggcgagcagaaaaaggccaucguggaccugcuguucaagaccaaccggaaagugaccgugaagcag
cugaaagaggacuacuucaagaaaaucgagugcuucgacuccguggaaaucuccggcguggaagaucgguucaacgccuc
ccugggcacauaccacgaucugcugaaaauuaucaaggacaaggacuuccuggacaaugaggaaaacgaggacauucugg
aagauaucgugcugacccugacacuguuugaggacagagagaugaucgaggaacggcugaaaaccuaugcccaccuguuc
gacgacaaagugaugaagcagcugaagcggcggagauacaccggcuggggcaggcugagccggaagcugaucaacggcau
ccgggacaagcaguccggcaagacaauccuggauuuccugaaguccgacggcuucgccaacagaaacuucaugcagcuga
uccacgacgacagccugaccuuuaaagaggacauccagaaagcccagguguccggccagggcgauagccugcacgagcaca
uugccaaucuggccggcagccccgccauuaagaagggcauccugcagacagugaagguggugggacgagcucgugaaagu
gaugggccggcacaagcccgagaacaucgugaucgaaauggccagagagaaccagaccacccagaagggacagaagaacag
ccgcgagagaaugaagcggaucgaagagggcaucaaagagcugggcagccagauccugaaagaacaccccguggaaaacac
ccagcugcagaacgagaagcuguaccuguacuaccugcagaaugggcgggauauguacguggaccaggaacuggacauca
accggcuguccgacuacgauguggaccauaucgugccucagagcuuucugaaggacgacuccaucgacaacaaggugcug
accagaagcgacaagaaccggggcaagagcgacaacgugcccuccgaagaggucgugaagaagaugaagaacuacuggcg
gcagcugcugaacgccaagcugauuacccagagaaaguucgacaaucugaccaaggccgagagaggcggccugagcgaac
uggauaaggccggcuucaucaagagacagcugguggaaacccggcagaucacaaagcacguggcacagauccuggacucc
cggaugaacacuaaguacgacgagaaugacaagcugauccgggaagugaaagugaucacccugaaguccaagcugguguc
cgauuuccggaaggauuuccaguuuuacaaagugcgcgagaucaacaacuaccaccacgcccacgacgccuaccugaacgc
cgucgugggaaccgcccugaucaaaaaguacccuaagcuggaaagcgaguucguguacggcgacuacaagguguacgacg
ugcggaagaugaucgccaagagcgagcaggaaaucggcaaggcuaccgccaaguacuucuucuacagcaacaucaugaac
uuuuucaagaccgagauuacccuggccaacggcgagauccggaagcggccucugaucgagacaaacggcgaaaccgggga
gaucgugugggauaagggccgggauuuugccaccgugcggaaagugcugagcaugccccaagugaauaucgugaaaaag
accgaggugcagacaggcggcuucagcaaagagucuauccugcccaagaggaacagcgauaagcugaucgccagaaagaa
ggacugggacccuaagaaguacggcggcuucgacagccccaccguggccuauucugugcugguggugggccaaaguggaa
aagggcaaguccaagaaacugaagagugugaaagagcugcuggggaucaccaucauggaaagaagcagcuucgagaagaa
ucccaucgacuuucuggaagccaagggcuacaaagaagugaaaaaggaccugaucaucaagcugccuaaguacucccugu
ucgagcuggaaaacggccggaagagaaugcuggccucugccggcgaacugcagaagggaaacgaacuggcccugcccucc
aaauaugugaacuuccuguaccuggccagccacuaugagaagcugaagggcucccccgaggauaaugagcagaaacagcu
guuuguggaacagcacaagcacuaccuggacgagaucaucgagcagaucagcgaguucuccaagagagugauccuggccg
acgcuaaucuggacaaagugcuguccgccuacaacaagcaccgggauaagcccaucagagagcaggccgagaauaucaucc
accuguuuacccugaccaaucugggagccccugccgccuucaaguacuuugacaccaccaucgaccggaagagguacacca
gcaccaaagaggugcuggacgccacccugauccaccagagcaucaccggccuguacgagacacggaucgaccugucucagc
ugggaggcgac
TRAC-sg 2 (T2) sequence: SEQ ID NO: 2
   gcugguacacggcaggguca
TRAC-sg 3 (T3) sequence: SEQ ID NO: 3
   cucucagcugguacacggca
TRAC-sg 4 (T4) sequence: SEQ ID NO: 4
   auuuguuugagaaucaaaau
TRAC-sg 6 (T6) sequence: SEQ ID NO: 5

   ucucucagcugguacacggc
B2M-sg 1(B1) sequence: SEQ ID NO: 6
   acucucucuuucuggccugg
B2M-sg 2(B2) sequence: SEQ ID NO: 7
   gaguagcgcgagcacagcua
B2M-sg 3(B3) sequence: SEQ ID NO: 8
   cgcgagcacagcuaaggcca
B2M-sg 4(B4) sequence: SEQ ID NO: 9
   ucacgucauccagcagagaa
B2M-sg 5(B5) sequence: SEQ ID NO: 10
   gcuacucucucuuucuggcc
B2M-sg 6(B6) sequence: SEQ ID NO: 11
   uuugacuuuccauucucugc
B2M-sg 7(B7) sequence: SEQ ID NO: 12
   cgugaguaaaccugaaucuu
B2M-sg8(B8) sequence: SEQ ID NO: 13
   cucgcgcuacucucucuuuc

The amino acid sequence of scFv of anti-TCRα/β: SEQ ID NO: 14 
QIVLTQSPAIMSASPGEKVTMTCSATSSVSYMHWYQQKSGTSPKRWIYDTSKLASGV
PARFSGSGSGTSYSLTISSMEAEDAATYYCQQWSSNPLTFGAGTKLELKGGGGSGGG
GSGGGGSGGGGSEVQLQQSGPELVKPGASVKMSCKASGYKFTSYVMHWVKQKPGQ
GLEWIGYINPYNDVTKYNEKFKGKATLTSDKSSSTAYMELSSLTSEDSAVHYCARGS
YYDYDGFVYWGQGTLVTVSA
 wherein the underlined part is the linker sequence.

The nucleotide sequence of CD8 alpha hinge region: SEQ ID NO: 15

accacgacgccagcgccgcgaccaccaacaccggcgcccaccatcgcgtcgcagcccctgtccctgcgcccagaggcgtgccgg
ccagcggcggggggcgcagtgcacacgaggggggctggacttcgcctgtgat

The nucleotide sequence of CD8 transmembrane domain: SEQ ID NO: 16 atctacatctgggcgcccttggccgggacttgtggggtccttctcctgtcactggttatcaccctttactgc

The nucleotide sequence of 4-11BB signal region: SEQ ID NO: 17

aaacggggcagaaagaaactcctgtatattcaaacaaccatttatgagaccagtacaaactactcaagaggaagatggctgtagctg
ccgatttccagaagaagaagaaggaggatgtgaactg

The nucleotide sequence of CD3 zeta: SEQ ID NO: 18

agagtgaagttcagcaggagcgcagacgcccccgcgtacaagcagggccagaaccagctctataacgagctcaatctaggacgaa
gagaggagtacgatgttttggacaagagacgtggccgggaccctgagatggggggaaagccgagaaggaagaaccctcaggaag
gcctgtacaatgaactgcagaaagataagatggcggaggcctacagtgagattgggatgaaaggcgagcgccggaggggcaagg
ggcacgatggcctttaccagggtctcagtacagccaccaaggacacctacgacgcccttcacatgcaggccctgccccctcgc

The amino acid sequence of the light chain variable region of scFv of anti-TCRα/β: SEQ ID NO: 19

QIVLTQSPAIMSASPGEKVTMTCSATSSVSYMHWYQQKSGTSPKRWIYDTSKLASGV
PARFSGSGSGTSYSLTISSMEAEDAATYYCQQWSSNPLTFGAGTKLELK

The amino acid sequence of the heavy chain variable region of scFv of anti-TCRα/β: SEQ ID NO: 20

EVQLQQSGPELVKPGASVKMSCKASGYKFTSYVMHWVKQKPGQGLEWIGYINPYN
DVTKYNEKFKGKATLTSDKSSSTAYMELSSLTSEDSAVHYCARGSYYDYDGFVYWG
QGTLVTVSA

The amino acid sequence of the linker for linking the light chain variable region and heavy chain variable region of scFv: SEQ ID NO: 21
GGGGSGGGGSGGGGSGGGGS

## Claims

1. A method for preparing a universal CAR-T cell targeting T-cell lymphoma cells, comprising:
1) obtaining T cells from a human donor having a healthy lymphatic system;
2) disrupting the following genomic regions in the T cells by gene editing technology:
(i) a TRAC genomic region; and
(ii) a B2M genomic region; and
3) stably expressing CAR molecules targeting T-cell lymphoma cell TCRα/βin the T cells.

2. The method according to claim 1, further comprising culturing the cells obtained from step 3) for an appropriate time to deplete T cells still expressing natural TCR.

3. The method according to claim 1 or 2, wherein the TRAC genomic region comprises the genomic region from positions 23016448 to 23016490 of human chromosome 14, and the B2M genomic region comprises the genomic region from positions 45003745 to 45003788 of human chromosome 15.

4. The method according to any one of claims 1-3, wherein the extracellular region of the CAR molecule targeting T-cell lymphoma cell TCRα/β is an scFv molecule binding to the extracellular region of T-cell lymphoma cell TCRα/β.

5. The method according to claim 4, wherein the CAR molecule comprises the following sequences linked by a linker:
the light chain variable region of the scFv: SEQ ID NO: 19, 
QIVLTQSPAIMSASPGEKVTMTCSATSSVSYMHWYQQKSGTSPKRWIYDTSKLAS
GVPARFSGSGSGTSYSLTISSMEAEDAATYYCQQWSSNPLTFGAGTKLELK; and
the heavy chain variable region of the scFv: SEQ ID NO: 20,

EVQLQQSGPELVKPGASVKMSCKASGYKFTSYVMHWVKQKPGQGLEWIGYINP
YNDVTKYNEKFKGKATLTSDKSSSTAYMELSSLTSEDSAVHYCARGSYYDYDGFVY
WGQGTLVTVSA.

6. The method according to claim 5, wherein the linker is an amino acid sequence comprising (G)n(S)m, and wherein n is a positive integer of 1-20, and m is a positive integer of 1-10.

7. The method according to claim 6, wherein the linker sequence is the sequence GGGGSGGGGSGGGGSGGGGS represented by SEQ ID NO: 21.

8. The method according to any one of claims 1-7, wherein the TRAC genomic region and the B2M genomic region are disrupted by homologous recombination, zinc finger nuclease gene editing technology, TALEN gene editing technology, or CRISPR/Cas gene editing technology.

9. The method according to claim 8, wherein the gene editing technology is CRISPR/Cas9 gene editing technology.

10. The method according to claim 9, wherein:
(i) the guide RNA (sgRNA) targeting the TRAC genome has a sequence selected from any one of SEQ ID NOs: 2-5; and/or
(ii) the guide RNA (sgRNA) targeting the B2M genome has a sequence selected from any one of SEQ ID NOs: 6-13.

11. The method according to claim 10, wherein the nucleotide sequences encoding the sgRNA and Cas9 are introduced into the T cells concurrently or separately.

12. The method according to claim 10 or 11, wherein the sgRNA is chemically modified.

13. The method according to claim 12, wherein the chemical modification comprises a 2'-O-methyl modification or an internucleotide 3'-thio modification.

14. The method according to claim 13, wherein the chemical modification is 2'-O-methyl modification on each of three bases at the 5' end and three bases at the 3' end of the sgRNA andinternucleotide 3'-thio modification on the three bases at the 5' end and three bases at the 3' end of the sgRNA.

15. The method according to any one of claims 12-14, wherein the sgRNA is co-introduced with an mRNA nucleotide sequence encoding Cas9 into the T cells by electroporation.

16. The method according to claim 15, wherein the electroporation condition is selected from any one of the following conditions: 150-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; and 250V, 0.5ms.

17. The method according to any one of claims 1-16, wherein the TRAC and B2M are individually knocked out with an efficiency of more than 90%, and concurrently knocked out with an efficiency of more than 75%.

18. The method according to any one of claims 1-17, wherein the nucleotide sequence encoding the CAR molecule targeting T-cell lymphoma cell TCRα/β is introduced into the T cells by viral transfection.

19. The method according to claim 18, wherein the virus is an adeno-associated virus (AAV) or a lentivirus.

20. The method according to any one of claims 1-17, wherein the nucleotide sequence expressing the CAR molecule is introduced into the TRAC gene locus of the T cell by homologous recombination, thereby the nucleotide sequence encoding the CAR molecule is expressed at the TRAC gene locus under the control of a TCR gene promoter.

21. The method according to any one of claims 1-20, wherein the cells obtained from step 3) are cultured for 3-21 days to deplete T cells retaining expression of natural TCR.

22. A universal CAR-T cell targeting T-cell lymphoma cells, wherein the CAR-T cell expresses CAR molecules targeting T-cell lymphoma cell TCRα/β.

23. The universal CAR-T cell according to claim 22, wherein the CAR molecule comprises the amino acid sequence as shown by SEQ ID NO: 14.

24. The universal CAR-T cell according to claim 22 or 23, wherein the CAR-T cell expresses at a low level or does not express one or more proteins selected from the group consisting of: TCRα/β, class 1 HLA protein, PD-1, TIM3, and LAG3.

25. A biological product comprising the universal CAR-T cell according to any one of claims 22-24.

26. The biological product according to claim 25, wherein 98% or more of the universal CAR-T cells are TCR negative.

27. Use of the universal CAR-T cell according to any one of claims 22-24 or the biological product according to claim 25 or 26 in the preparation of a medicament for treating T-cell lymphoma.

28. A method for treating T-cell lymphoma, comprising administering a therapeutically effective amount of the universal CAR-T cell according to any one of claims 22-24 or the biological product according to claim 25 or 26 to a subject suffering from T-cell lymphoma.
